Europäisches Patentamt

European Patent Office

Office européen des brevets

(11) Publication number: **0 164 435 B1**

# EUROPEAN PATENT SPECIFICATION

(45) Date of publication of patent specification: 24.07.91   (51) Int. Cl.⁵: **A61F 5/44**, A61F 13/15

(21) Application number: **84106820.8**

(22) Date of filing: **14.06.84**

(54) Incontinent garment with elasticized pouch.

(43) Date of publication of application:
**18.12.85 Bulletin 85/51**

(45) Publication of the grant of the patent:
**24.07.91 Bulletin 91/30**

(84) Designated Contracting States:
**DE FR GB NL**

(56) References cited:
EP-A- 0 023 804    EP-A- 0 091 412
EP-A- 0 098 512    CH-A- 577 281
CH-A- 620 816     GB-A- 2 011 778
GB-A- 2 026 323    US-A- 2 965 102
US-A- 3 371 668    US-A- 4 050 462

(73) Proprietor: **KIMBERLY-CLARK CORPORATION**
**401 North Lake Street**
**Neenah Wisconsin 54956(US)**

(72) Inventor: **Damico, Joyce A.**
**1219 Green Acres Lane**
**Neenah, Wisconsin(US)**
Inventor: **Weber, Rebecca J.**
**757 Oak Street**
**Neenah, Wisconsin(US)**

(74) Representative: **Patentanwälte Grünecker,**
**Kinkeldey, Stockmair & Partner**
**Maximilianstrasse 58**
**W-8000 München 22(DE)**

## Description

The invention relates to a disposable absorbent pad.

Such pads are to be used to receive discharge from the body, particularly as disposable incontinent garments for adult active ambulatory persons. Many articles utilized as incontinent products for adults and other ambulatory persons have been found unsatisfactory as they are bulky and ineffective. Many such garments were formed by folding up flat sheets into a diaper-like structure which was bulky, particularly in the crotch portion. It further had a tendency to become dislodged during activity.

From US-A-4 253 461 a disposable absorbent brief is known which comprises an impervious back-sheet, a pervious body-side liner joined to said back-sheet at their edges, an absorbent liner between the body-side liner and the back-sheet, and elastic portions generally centered at peripheral portions of said pad. The width of the pad in its effective area amounts to 25% of the extended total pad length; the length of the elasticized area when the elastic is in extended condition amounts to 32% of the extended total pad length; the shortening of the total pad length when the elastic relaxes is 16% of the extended total pad length.

While successful in containing discharges for incontinent persons, the pad known from US-A-4 253 is bulky and expensive. The pad is required to be large in order to wrap around the area of the patient from the waist to below the hip, and form a tight leg seal which is generally necessary to prevent leakage when a person is lying down. Diaper-like garments of this kind are not desirable for active persons as they are bulky and interfere with wearing of ordinary clothes. Further, the large amount of material utilized makes these garments expensive.

It has been proposed that smaller disposable articles be used for incontinent adults such as the article disclosed in US-A-4 182 334, in which a specially-folded absorbent article is utilized as a containment device for discharges, particularly from adults. The device could be held in place by wearing inside an undergarment or with a suspension means.

A further device for adult incontinence is disclosed in US-A-4 315 508 in which a suspension system for holding pads in the perineal region in order to minimize leakage is shown.

US-A-4 337 771 discloses a generally rectangular pad having elasticized edges. The pad may have two or four edges elasticized. The pad is designed to be worn in a diaper-like manner by wrapping around the body. Devices such as this are bulky and uncomfortable, having a great amount of material in the crotch area. Further, they are not comfortable or suitable for active movement.

The diaper art for infants is well-developed and discloses garments that are designed to be worn in a self-supporting manner by wrapping around the waist and being fastened.

However, there remains a need for a garment that may be worn during normal activities and will be very leak-resistant. The leak-resistance is particularly desirable when the person wearing the garment is active or when the person wearing the garment sits on the garment when wet.

There is particular need for a garment that is low in cost, disposable, able to be worn discreetly under ordinary clothes, and resistant to leaks when the wet garment is worn in a sitting position.

An object of this invention is to overcome disadvantages of the prior art.

An object of this invention is to produce an incontinent garment that will be effective but not visible under street clothes.

A further object of this invention is to produce a garment that will resist leaks during a person's activities.

It is another object of this invention to produce a garment that will protect from leakage when it is sat upon when wet.

Another object of this invention is to produce a low-cost incontinent garment.

An additional object of this invention is to produce an incontinent garment that will allow comfortable wear.

It is another additional object of this invention to produce an incontinent-care garment that will allow active movement while being worn.

These and other objects of the invention are accomplished by providing a disposable, absorbent pad having the features of claim 1.

In a preferred embodiment for adults, the width is about 28 percent of the length and the length of the elasticized area to the overall pad length, is between about 40 and about 56 percent. The pad of the invention may be held in place by support devices or be worn inside an undergarment.

Embodiments of the invention are shown in the drawings, wherein:

Figure 1 is a plan view of a garment of the invention in extended condition with the body-contacting portion facing the viewer.

Figure 2 is a cross-section view of the invention garment taken on section line 2 - 2 of Figure 1.

Figure 3 is a cross-sectional view of the invention garment taken on line 3 - 3 of Figure 1.

Figure 4 is a perspective view of garment of the invention when in a position such as it assumes during use.

Figure 5 illustrates the garment of the invention when worn with a suspension system.

Figure 6 is a plan view of the garment of the invention viewed when the garment is positioned in a manner similar to the shape when in used.

Figure 7 is a side plan view of a person wearing the garment of the invention.

Figure 8 is a cross-sectional view of the garment in use taken along line 8 - 8 of Figure 7.

Figure 9 is a plan view of an alternative garment of the invention without the baffle feature.

Figure 10 is a sectional view on line 10 - 10 of Figure 9.

## MODE FOR CARRYING OUT THE INVENTION

The invention has numerous advantages over the prior art. The device of the invention is not bulky and allows activities of ambulatory people wearing the device without embarrassment. Further the device has a very low tendency to leak. The device further is low in cost as it is formed of a rectangular shape thereby allowing full use of raw materials. Complicated shapes requiring leg cutouts or other intricate shapes require wasting of material in making these cutouts. The device is easy to make as it does not require folding, which contributes to low-cost production. The device is more comfortable as it produces a pouch effect such that the material contained in the device is less likely to be in contact with the perineal region. Further, the organs of the perineal area are not rigidly confined thereby causing discomfort. A further advantage, particularly with the baffled garment, is that the garment is less likely to leak when sat upon when wet than other garments. The garment's elastic portion at the edges of the perineal region further makes the wearer feel more secure and less concerned about staining or leaking. These and other advantages will become clear from the detailed description that is given below.

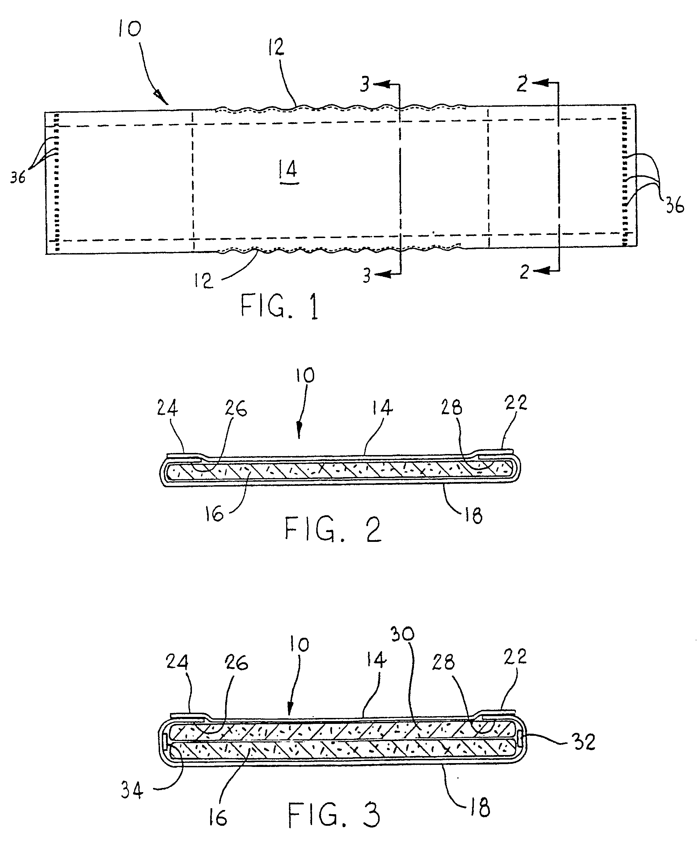

The pouch-pad garment indicated as 10 in Figure 1 is shown in extended condition. By this it is meant that the pad elastic is fully extended so the pad is flat. The pad 10 as seen from top view comprises a generally rectangular member having a shirred edge 12, where the elastic is attached to the inner portion of the polymer sheet at the edge of the garment and a body facing pervious liner 14 overlaying the pad and impervious back sheet member. As indicated in the cross-section view, Figure 2, taken at line 2 - 2 of Figure I, the pad comprises the body facing member 14 of pervious material, inner lower pad member 16, and impervious polymer member 18, which is wrapped around pad member 16 and adhered to facing member 14 at 22 and 24. This structure provides that the body facing porous sheet covers the impervious member at the edges. The overlap of the backing member 18 at 22 and 24 creates a baffle area of impervious sheet 26 and 28 that aids in containment of bodily discharges when the device is in use. The center portion of the pad 10, as illustrated in Figure 3, contains an additional layer of absorbent material 30, and further has elastic members 32 and 34 extending along the longitudinal edges of the rectangular pad. While the devices are shown with two layers of padding it is, of course, within the invention to utilize any number of desired layers or to use unitary pads that are formed with a thicker portion in the middle. However, for simplicity of formation, the use of a thickened center portion formed by additional layers of absorbent padding is simple and does not result in waste of material.

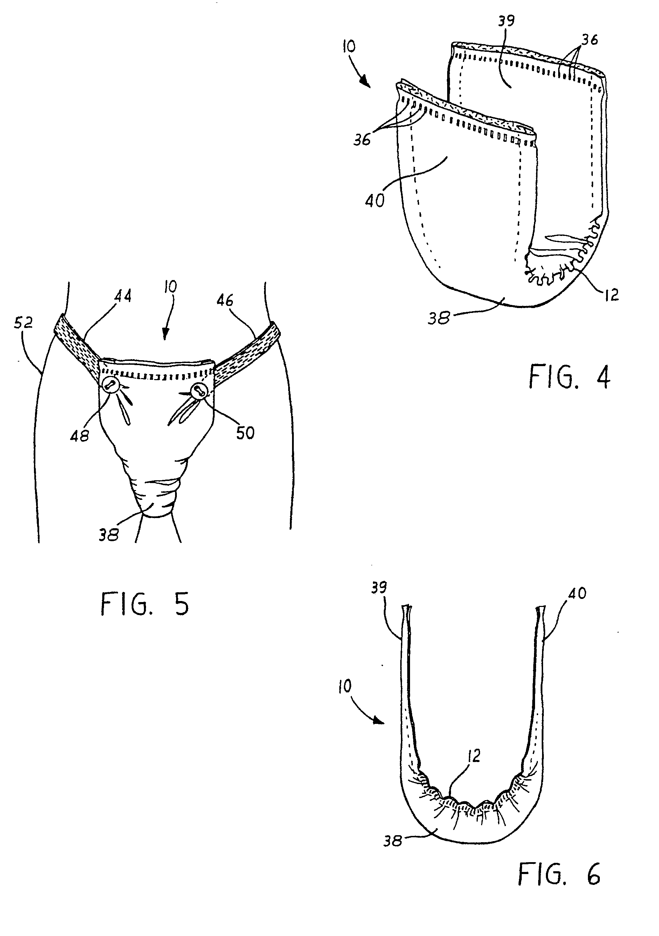

Figure 4 is a view of the pouch pad of the invention 10 in a relaxed condition and generally in the form assumed when it is worn. The spots 36 are places where the impervious back sheet pad, and body-side face sheet were ultrasonically sealed together. The device has a pronounced pouch 38. The elasticized area 12 has assumed a bunched shape and is well raised above the bottom of the pouch 38. The non-elasticized areas 39 and 40 extend up in front of and behind the person wearing the garment, provide extra absorbent material that may be needed when sitting and also may be utilized to position fasteners to keep the garment in place.

Figure 5 represents a garment in accordance with the invention held in place by the suspension system disclosed in U.S. Patent 4,315,508, in which elastic members 44 and 46 extend up over the hips of the wearer and provide an upward force onto the garment 10. The straps 44 and 46 are terminated by button-like devices 48 and 50 that extend through holes in the garment 10. It is also possible that the garment may be held in place by being worn inside a tight-fitting undergarment.

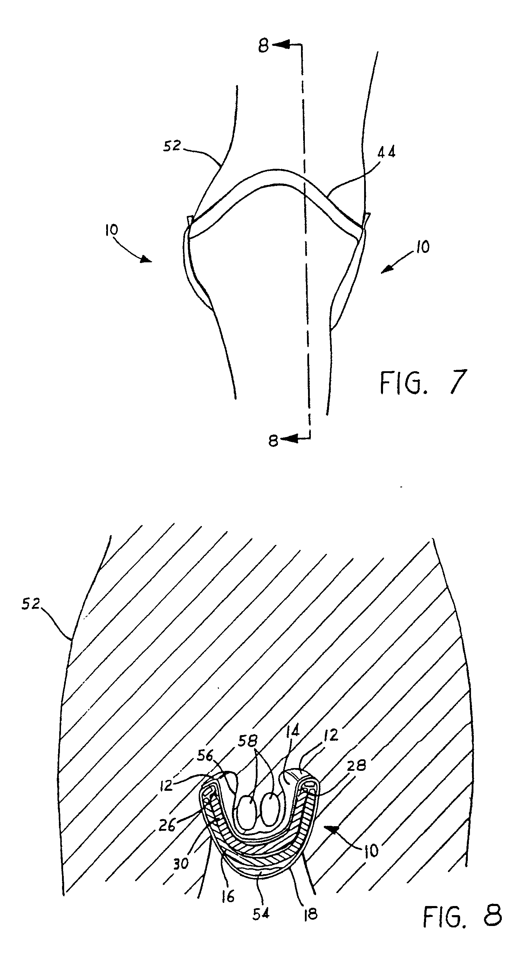

Figure 6 is a sideview of the garment 10 in which the pouch-like effect of the garment is clearly illustrated. In the invention garment the depth between the elastic edge and the bottom of the garment in the center portion may be about 2 inches (50,8mm) in a garment of overall size of 25" x 7" (635 x 177,8mm). As illustrated in Figures 7 and 8, the garment of the invention 10 is worn by a person 52. Figure 8 is a cross-sectional view on line 8 - 8 of Figure 7. The garment of the invention 10 is illustrated and the benefits of the pouch formed in the invention are illustrated. The device with the impervious outer shield 18 and the swollen absorbent materials 16 and 30 has retained urine in the absorbent and also some urine 54 which has pooled. As can be seen from the illustration, the baffles 26 and 28 will prevent the overflow of urine 54 during activities. Further, the baffles 26 and 28 will direct the flow of urine along the edge of the pad rather than over the edge when pad is sat upon or compressed by movement. The elastic edges 12 hold the edges higher than the lower center portion when it is worn and further provide a pouching effect for comfortable fit around the scrotum 56 and testicles 58 of the wearer 52. Further, the elastic members holding the device in place without discomfort provide a feeling of security to the wearer that leakage will not leak during activities. While shown with a male wearer, the device also, of course, is suitable for women.

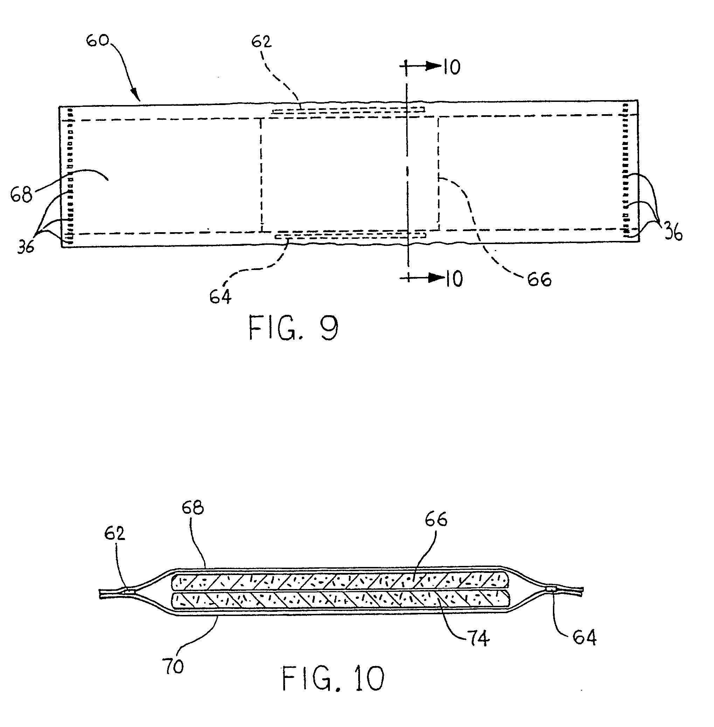

Figures 9 and 10 illustrate a less preferred embodiment of the invention in which baffles are not present, indicated generally as 60. In this embodiment the baffle is not present. The pervious body side liner 68 and the impervious backing sheet 70 are joined at the sides, and the elastics 62 and 64 are located between the pervious liner 68 and backing sheet 70. The device is illustrated with two absorbent filler sheets (66 and 74) similar to that illustrated in the preferred embodiment. However, as before stated, the number filler sheets and their thickness may be varied although the device generally should have more absorbence in the pouch portion.

It is theorized that the device of the invention is particularly advantageous over prior art devices in that the amount of stress that is utilized in the elastic is very high thereby shortening the edges of the elasticized portion a great deal, causing the pouched effect. It is theorized that this high degree of contraction is suitable for the invention garment in that no leg seals are being formed as in diaper-type garments. A high degree of contraction in a garment forming leg seals will cause red marks and difficulty in circulation whereas in a device such as the instant invention the elastic acts primarily to shape the garment and the force of the elastic is not applied directly to the body or around the legs of the person wearing the garment. However, the elastic does contact the body of the wearer and may exert slight pressure, contributing to a feeling of security in wearing the garment.

It has been found that the pad of the invention has specific dimensions and properties which are believed interrelated to result in the particularly desirable garment of the invention. The pad of the invention is generally narrower than previous garments utilized for absorption of body excretions. The width of the pad is generally found to be between about 20 - 35 percent of the extended pad length in order to have good wearing characteristics and desired pouch shape in addition to providing enough absorbent material. It has been found that a particularly preferred article for use in adult incontinence protection has a width of about 28 percent of the length dimension for comfortable wearing and large capacity for holding of excretions. Another dimension which has been found to be of importance with the invention is the relationship of length of the elasticized area to the total pad length. The elasticized material is located on the long side of the rectangular pad and is generally centered on the long side so as to leave equal non-elasticized portions at each end. It has been found suitable that the length of extended elasticized material be between about 25 and 60 percent of the total pad length in order to create the desired pouch effect. The particularly preferred form for use in adult incontinent care has a length of the elasticized area of between about 40 - 56 percent of the total pad length for good pouch formation and comfort to the wearer. A property that is also important in the instant invention is the amount of foreshortening of the pad caused by the elastic. As stated above, the elastic in the instant pad is highly stretched when attached and therefore a relatively great amount of shortening takes place. It is, of course, possible that thicker elastic stretched by a smaller amount could cause the same amount of foreshortening and the type of elastic is not believed critical. The important feature is the amount of foreshortening which will cause the desired pouch effect. The relaxed pad is generally measured by placing the pad with the body-side portion facing upward and gently flattening it by hand prior to measurement. The amount of pressure required for flattening is no more than that applied by a two-to-three-pound (0,9 - 1,3kg) book of 9-1/2" x 11" (241,3 x 279,4mm) size laying on the pad. The amount of foreshortening of the entire pad length caused by the shrinkage of the elastic is suitably between about 15 - 37 percent of the overall extended pad length. The preferred amount is between about 20 - 32 percent of extended pad length for creation of a good pouch that is readily moldable to body contours.

The pads of the invention may be made any desirable size as long as the relationship of the dimensions is within the above-listed parameters. However, it may be stated that for a typical adult incontinent garment the width is about 7 inches and the overall extended pad length is about 25 inches (635mm). However, generally the pad size ranges for extended adult garments would be considered in the range of between about 18 (457,2mm) and about 29 inches (736,6mm) for length and about 5 (127mm) and about 8 inches (203,2mm) for width. It is, of course, possible that when the garments are used for bodily excretion not related to incontinence, i.e., wounds of the knee or head, that smaller dimensions would be used. For instance, a pad suitable for use with an elbow may be about 5 inches X 20 inches (127 x 508mm). Head bandage garments are of similar size to those for incontinent care garments or may be slightly smaller such as 6 inches by 27 inches (152,4mm by 685,8mm).

The impervious backing sheet of the invention may be any suitable flexible impervious film. Typical of such materials are films of polyvinyl chloride, polyesters, and other polymer materials. Preferred materials are the linear polyolefin films such as polyethylene and polypropylene as they are low cost and quiet. The backing sheet may be of laminated construction as long as one of the laminated sheets is impervious.

The body-side liner material may be any porous material that is generally nonwetting and porous to fluids. Typical such materials are nonwoven webs of cellulose materials, nonwoven webs of synthetic fibers such as polyester, perforated polymer films, and porous woven materials. A preferred material is spunbonded polypropylene which is a material formed by meltblowing of extruded polypropylene. The material is strong and does not greatly deteriorate in strength when wet. While the body-side liner is illustrated as a rectangular piece of the same size as the garment, it may be that in some manufacturing techniques it would be desired to wrap the pervious material entirely around the garment. In that case only the portion over the absorbent would be necessary.

The absorbent material forming the absorbent portion of the device of the invention may be any of many well-known absorbent materials such as cellulose fibers ordinarily referred to as fluff or mixtures of cellulose fibers and meltblown artificial fibers. Further, it is within the invention to utilize other absorbent materials such as superabsorbents or artifical sponge material. The preferred material has been found to be blends of polypropylene and cellulose fibers that are formed by blowing cellulose fibers into polypropylene fiber during formation such as those formed by the process of U.S-A-4,100,324. It is further possible to use combinations of batts of different material and to use any desired number of layers. Generally the device would be formed with a thicker absorbent filling in the pouch portion.

The device of the invention may be held in place by numerous means. As illustrated in the drawings, the device is shown held up with a suspension elastic that is terminated by buttons which fit through holes in the garment. Other suspension devices may be utilized such as elastics with clips which fasten onto the device or adhesive connections or VELCRO® fasteners. Further, rather than being held in suspension, the device may be utilized with tight-fitting undergarments. These garments may be either specially designed garments or the pouch pad may be worn inside ordinary underwear of the brief style.

The device of the invention is of particular advantage in that it is not greatly noticeable under ordinary clothing. It is noted that the device ordinarily will not extend above the navel of the wearer in the front and also reaches to a similar low level in the back. Further, as was illustrated in the drawings, the device generally has less padding in what will be the upper portion when worn. The absorbent in the upper areas generally would be utilized when the person wearing the garment sat down and fluid then would be forced into the upper portions.

While the invention is illustrated in Figures 1 through 3 with the baffles (26 and 28) located beneath the pervious body-side lining 14, it would also be within the invention to place the baffles on the outer surface. This would have the disadvantage that the plastic would be in contact with the skin such that it would be likely to be less comfortable. However, there might be somewhat less chance of wicking of the fluids from the garment as the pervious material does not extend to the edge. The baffle is effective whether it is below or above the pervious liner.

The following example is intended to be illustrative of a method of forming a pouch pad in accordance with the invention. This is a laboratory technique of making the device and, of course, would be automated in commercial production. However, it will be clear from the example below, that the device is easily constructed without intricate cutting or folding required.

EXAMPLE

A piece of polypropylene sheeting of about .7 mils (0,0178mm) thickness is cut to a size of 9 inches by 27 inches (228,6mm by 685,8mm). sheet is taped to a laboratory table in an extended flat position. An absorbent member is cut to a size of about 7 inches by 14 inches (177,8mm by 355,6mm) and centered onto the polypropylene sheet. Another sheet of absorbent material is cut to a size of about 7 inches by 25 inches (177,8mm by 635mm) and this is also centered onto the polypropylene sheet to leave about an inch on each longitudinal edge and about an inch on each of the shorter edges exposed. The absorbent sheet was a sheet formed of a combination of meltblown polypropylene and cellulose fibers. Each pad was about 3/16 of an inch (4,762mm) thick when dry. Next two-sided tape of about 1 inch (25,4mm) width was placed on the exposed longitudinal edges. Next the elastic pieces for the edge were cut. These were natural rubber about 1/4 inch (6,35mm) wide and about 7 mil (0,1778mm) thickness. In a relaxed condition, these pieces were 4-1/2 inches (114,3mm) long. The pieces were stretched 14 inches (355,6mm) and stuck to the tape immediately adjacent the absorbent material and centered in the longitudinal portions. The exposed longitudinal edges having two-sided tape on them are then folded over and adhered to the pad. This has the effect of forming the baffle and also concealing the tape from contact with the skin in the finished article. Next a 1/2 inch (12,7mm) wide portion of two-sided tape was applied to the marginal longitudinal edge of the pad. This tape is therefore located on the outside of the folded baffle. Next the body-side liner of spunbonded polyester was cut to a size of 7 inches by 25 inches (177,8mm by 635mm) and laid on top of the pad and sealed at the edges to the tape. The pad was then cut lose from the laboratory table and the end portions were sealed with an ultrasonic gun. The excess polypropylene sheet on the end portions was cut off such that the backing member and the pad and body-side material were the same length.

After being cut free of the laboratory table the pad was of course shortened by the elastic which attempted to return to its original length. The length of the pad after shortening was about 18-1/2 inches (469,9mm). This length is measured after laying the pad flat and weighting the elastic portion with a book of about 2-1/2 pounds (1,13kg) weight and about 11 inches (279,4mm) long. This shortening of the elastic had the effect of creating the pouch and forming the pouch pad of the invention. Pads formed such as in this example were tested on several ambulatory people having incontinence problems and found to have a very low level of leakage. Further, the pouch pad was found to be comfortable to wear, giving feeling of security and was particularly resistant to leaks when it was sat upon when wet.

While the invention has been discussed primarily with respect to the use of the pad of the invention as an incontinent garment, the pouch pad of the invention has other uses, particularly in the area of use as bandaging or as a garment to prevent chaffing and irritation of the skin in areas of abrasion. When used in this manner, it may prevent bed sores or blistering. For instance, the pad when made in the proper size finds utilization as a bandage for a knee wound. When used in this manner, a two-sided tape with cover means may be placed on to the end of the pad so that after it is wrapped around the knee it may be sealed. Alternatively, the device may be taped in place. Also, the pad of the invention may be used as a prophylactic measure to prevent bed sores. Such a use would be achieved when properly-sized garments are wrapped around the bodily protrusions such as the heels, elbows, and knees. The device further finds utility as a bandage device for head wounds as the pouch shape adapts itself to being held in place upon the head.

These other uses of the garment may find particular utility in the nursing home environment as it would make the garment a multi-purpose unit that could be used in the same size for head bandages and incontinent care. Further, sizes suitable for wrapping knees also would be suitable for use in incontinent care of incontinent children or small adults. This would be a savings in the hospital and nursing home environment as fewer bandaging and care devices would be necessary.

The above description is intended to be descriptive and not exhaustive of the possibilities of the invention. For instance, while the invention is disclosed with utilization of natural rubber elastics, it is possible that the device could be formed with elastics of self-adhesive rubbers or other types of artificial rubber. Further, while disclosed with a plain polymer sheet surface, it also would be within the invention to provide the article with a decorated finish or bond the polymer covering material to a fabric surface which would provide better feel to the patient and quiet the crinkling of some polymer materials. It is noted that generally polypropylene films are relatively quiet when worn at body temperatures, but at lower temperatures there is more tendency to crinkle. Addition of a fibrous or cloth covering to the polypropylene would make the garment more esthetically pleasing prior to use.

**Claims**

1. A disposable, absorbent pad (10,60) comprising an impervious backsheet (18,70), a pervious body-side liner (14,68) joined to said backsheet (18,70) at their edges, an absorbent liner (16,66,74) between the body-side liner (14,68) and the backsheet (18,70), elastic portions (32,34,62,64) generally centered at peripheral portions of said pad, the length of the elasticized area when the elastic is in extended condition being between about 25% and about 60% of the total pad length and the shortening of the total pad length when the elastic relaxes being between about 15% and about 37% of the extended length, said backsheet (18,70), said body-side liner (14,68) and said absorbent liner (16,66,74) being generally rectangular in shape, wherein said pad width is between about 20% and about 35% of the extended total pad length, and said elasticized portions (32,34,62,64) are arranged on the long edges of the rectangle adjacent the absorbent liner (16,66,74) in order to hold the long edges of the absorbent liner (16,66,74) higher than the lower center portion, thus forming a pouch (38), when the pad is bent by contraction of the elastic.

2. The pad of claim 1 **wherein** said impervious backsheet (18) comprises impervious portions (22,24) overlapping a part of the body-side surface of the pad.

3. The pad of claim 2 **wherein** the impervious portion (22,24) overlapping said pad (10,60) is covered by said body-side liner (14).

4. The pad of claim 1 **wherein** the long edge portions of said impervious back sheet (18) are folded so as to cover a portion of the edge of the absorbent pad (10,60).

5. The pad of claim **1 wherein** said shortening of the pad (10,60) when the elastic relaxes is between about 20 and 32 percent.

6. The pad of claim 1 **wherein** said length of elasticized area to pad length is between about 40% and about 56% of total pad length.

7. The pad of claim 1 having a width of about 28% of the length of the extended elasticized portion.

8. The pad of claim 1 **wherein** the depth of the pouch (38) is about 50 mm (2 inches) below the elastic sides.

9. The pad of claim 1 being held in place by elastic supports (44,46).

10. The pad of claim 1 being held in place by an undergarment.

## Revendications

1. Garniture absorbante jetable (10, 60) comprenant une feuille de doublure imperméable (18, 70), une couche perméable en contact avec le corps (14, 68) réunie à ladite feuille de doublure (18, 70) le long de ses bords, une couche absorbante (16, 66, 74) entre la couche en contact avec le corps (14, 68) et la feuille de doublure (18, 70), des parties élastiques (32, 34, 62, 64) centrées généralement sur des parties périphériques de ladite garniture, la partie présentant un élastique pouvant atteindre, lorsque l'élastique est tendu, une longueur allant d'environ 25% à environ 60% de la longueur totale de la garniture, et la longueur totale de la garniture pouvant être, lorsque l'élastique se resserre, réduite à une longueur d'environ 15% à environ 37% de la longueur en condition d'extension, ladite feuille de doublure (18, 70), ladite couche en contact avec le corps (14, 68) et ladite couche absorbante (16, 66, 74) ayant généralement une forme rectangulaire, dans laquelle la largeur de ladite garniture est comprise entre environ 20% et environ 35% de la longueur totale de la garniture en condition d'extension, et dans laquelle lesdites parties présentent un élastique (32, 34, 62, 64) sont prévues sur les bords longs du rectangle à côté de la couche absorbante (16, 66, 74) afin de maintenir les bords longs de la couche absorbante (16, 66, 74) surélevés par rapport à la partie centrale abaissée, en formant ainsi une poche (38) lorsque la garniture est incurvée par contraction de l'élastique.

2. Garniture selon la revendication 1, dans laquelle ladite feuille de doublure imperméable (18) comprend des parties imperméables (22, 24) recouvrant une partie de la surface en contact avec le corps de la garniture.

3. Garniture selon la revendication 2, dans laquelle la partie imperméable (22, 24) recouvrant ladite garniture (10, 60) est couverte par ladite couche en contact avec le corps (14).

4. Garniture selon la revendication 1, dans laquelle les parties des bords longs de ladite feuille de doublure imperméable (18) sont pliées de manière à couvrir une partie du bord de la garniture absorbante (10, 60).

5. Garniture selon la revendication 1, dans laquelle ladite réduction de la garniture (10, 60) lors de la détente de l'élastique est comprise entre environ 20 pour cent et 32 pour cent.

6. Garniture selon la revendication 1, dans laquelle ladite longueur de la zone présentant un élastique par rapport à la longueur de la garniture est comprise entre environ 40% et environ 56% de la longueur totale de la garniture.

7. Garniture selon la revendication 1 ayant une largeur égale à environ 28% de la longueur de la partie présentant un élastique, lorsque ce dernier est tendu.

8. Garniture selon la revendication 1, dans laquelle la profondeur de la poche (38) est d'environ 50 mm (2 pouces) en-dessous des côtés présentant un élastique.

9. Garniture selon la revendication 1 maintenue en place par des supports élastiques (44, 46).

10. Garniture selon la revendication 1 maintenue en place par un sous-vêtement.

## Patentansprüche

1. Wegwerfbare, absorbierende Einlage (10, 60) mit einer undurchlässigen Rückseite (18, 70), einer mit der Rückseite (18, 70) an ihren Kanten verbundenen, durchlässigen, körperseitigen Bedeckung (14, 68), einer absorbierenden Bedeckung (16, 66, 74) zwischen der körperseitigen Bedeckung (14, 68) und der Rückseite (18,70), elastischen Bereichen (32, 34, 62, 64), die im wesentlichen mittig an Umfangsbereichen der Einlage angeordnet sind, wobei die Länge der elastisch gemachten Fläche, wenn das elastische Mittel in ausgestrecktem Zustand vorliegt, zwischen etwa 25 % und etwa 60 % der Gesamtlänge der Einlage ist, und die Verkürzung der Gesamtlänge der Einlage, wenn das elastische Mittel sich entspannt, zwischen etwa 15 % und etwa 37 % der ausgestreckten Länge beträgt, wobei die Rückseite (18, 70), die körperseitige Bedeckung (14, 68) und die absorbierende Bedeckung (16, 66, 74) eine im wesentlichen rechteckige Form aufweisen, wobei die Breite der Einlage zwischen etwa 20 % und etwa 35 % der ausgestreckten Gesamtlänge der Einlage beträgt, und die elastisch gemachten Bereiche (32, 34, 62, 64) an den langen Kanten des Rechteckes in der Nähe der absorbierenden Bedeckung (16, 66, 64) angeordnet sind, um die langen Kanten der absorbierenden Bedeckung (16, 66, 74) höher als den tieferen Mittelbereich zu halten, um dadurch eine Tasche (38) zu formen, wenn die Einlage durch die Kontraktion der elastischen Mittel gebogen wird.

2. Einlage nach Anspruch 1, wobei die undurchlässige Rückseite (18) undurchlässige Bereiche (22, 24) umfaßt, die einen Teil der körperseitigen Oberfläche der Einlage überlappen.

3. Einlage nach Anspruch 2, wobei der die Einlage (10, 60) überlappende, undurchlässige Bereich (22, 24) durch die körperseitige Bedeckung (14) abgedeckt ist.

4. Einlage nach Anspruch 1, wobei die langen Kantenbereiche der undurchlässigen Rückseite (18) derart gefaltet sind, daß sie einen Bereich der Kante der absorbierenden Einlage (10, 60) überdecken.

5. Einlage nach Anspruch 1, wobei die Verkürzung der Einlage (10, 60), wenn die elastischen Mittel sich entspannen, zwischen etwa 20 und 32 % liegt.

6. Einlage nach Anspruch 1, wobei die Länge der elastisch gemachten Fläche zur Länge der Einlage zwischen etwa 40 % und etwa 56 % der Gesamtlänge der Einlage beträgt.

7. Einlage nach Anspruch 1, die eine Breite von etwa 28 % der Länge des ausgestreckten, elastisch gemachten Bereichs aufweist.

8. Einlage nach Anspruch 1, wobei die Tiefe der Tasche (38) unter den elastischen Seiten etwa 50 mm (2 inch) beträgt.

9. Einlage nach Anspruch 1, die durch elastische Träger (44, 46) an Ort und Stelle gehalten wird.

10. Einlage nach Anspruch 1, die durch Unterwäsche an Ort und Stelle gehalten wird.

FIG. 1

FIG. 2

FIG. 3

FIG. 4

FIG. 5

FIG. 6

FIG. 7

FIG. 8

FIG. 9

FIG. 10